# EUROPÄISCHE PATENTSCHRIFT

(11) **EP 0 547 385 B1**
(45) Veröffentlichungstag und Bekanntmachung des Hinweises auf die Patenterteilung: **01.05.1996**
(21) Anmeldenummer: 92119590.5
(22) Anmeldetag: 17.11.1992
(51) Int. Cl.: B07C 5/00, B07C 5/34, G01N 1/00, G01N 35/02, G01N 27/62

(54) **Verfahren und Vorrichtung zur Fremdstoffinspektion von wiederverwendbaren Gefässen**
Method and apparatus for the inspection of foreign matter from reusable vessels
Procédé et dispositif pour l'inspection de corps étrangers de récipients réutilisables

(30) Priorität: 18.11.1991 DE 4137912
(43) Veröffentlichungstag der Anmeldung: 23.06.1993
(73) Patentinhaber: KHS Maschinen- und Anlagenbau Aktiengesellschaft, D-47057 Duisburg (DE)
(72) Erfinder: Kwade, Martin, Dr., W-4600 Dortmund 50 (DE); Knabe, Uwe, W-4755 Holzwickede (DE)

(56) Entgegenhaltungen:
- US-A- 4 208 372
- US-A- 4 858 767
- US-A- 4 858 768

## Beschreibung

Die Erfindung bezieht sich auf ein Verfahren und eine Vorrichtung zur Fremdstoffinspektion von wiederverwendbaren Gefäßen, insbesondere Kunststofflaschen, die in einer Inspektionsmaschine nacheinander einer Überprüfung unterzogen werden, wobei ein Teil der in dem Gefäß vorhandenen Luft von in den Gefäßhohlraum einführbaren Meßsonden angesaugt und einer Analyse unterworfen wird, wie beispielsweise aus US-A-4 858 767 bekannt.

Insbesondere in der Getränkeindustrie werden wiederverwendbare Kunststofflaschen eingesetzt, die vor der eigentlichen Flaschenreinigung auf mißbräuchliche Verunreinigungen mit anderen Medien, Rückständen von Fremdstoffen wie Benzin, Lösungsmittel, Alkohol, Altöl und anderen Mitteln untersucht werden müssen. Das verwendete Analysesystem in einer solchen Inspektionsmaschine erkennt Flaschen, bei denen sich die Flaschenatmosphäre vom Getränkearoma bzgl. der chemischen Zusammensetzung unterscheidet. Kontaminierte Flaschen werden dann mit Hilfe einer speziellen Vorrichtung aus dem Produktionsfluß ausgeschleust. Die Ausscheidung erfolgt zweckmäßig vor der Reinigung mit dem Vorteil, daß die in der Reinigungsmaschine verwendete Waschlauge nicht zusätzlich mit unerwünschten Substanzen belastet wird. Die verwendete Inspektionsmaschine besteht im wesentlichen aus einem Einlaufstern, einem drehbaren Meßkarussell und einem Auslaufstern. Die zu prüfenden Flaschen gelangen nach dem Einlauf in das aus mehreren Meßstellen bestehende Meßkarussell. An diesem befinden sich mehrere Meßsonden mit vertikal verschiebbaren Flaschenhalterungen. Das Eintauchen der Sonden wird dadurch realisiert, daß die Flaschen während ihrer Rotationsbewegung über eine Kurvenbahn angehoben und der Meßsonde zugeführt werden. Es ist aber auch denkbar, die Meßsonde verschiebbar auszubilden und in die auf konstantem Niveau geführten Flaschen abzusenken.

Alle Meßsonden sind über entsprechende Verbindungsleitungen mit einem zentrisch auf der Karussellachse angeordneten Umsteuerungssystem verbunden. Sobald sich eine Flasche in der korrekten Meßposition befindet, wird die Ansaugleitung zu dieser Flasche für eine Taktzeit mit dem Analysesystem verbunden und die eigentliche Messung durchgeführt. Nach Beendigung der Messung wird die betreffende Flasche wieder abgesenkt und vom Auslaufstern übernommen. Detektierte Flaschen werden dabei aus dem Produktionsfluß ausgesondert und auf ein separates Sammelband transportiert.

Aus der US-A-4 858 767 ist es bekannt, auf dem Karussell entsprechend der Anzahl der Meßsonden entsprechende Meßsysteme vorzusehen, die jeweils unabhängig voneinander die darunter befindliche Flasche überprüfen. Eine solche Ausgestaltung erfordert eine Vielzahl von empfindlichen Meßsystemen, die einen entsprechenden Platzbedarf und damit einen relativ großen Drehkreis und im übrigen aufgrund der Vielzahl auch einen entsprechend höheren Steuerungs- und Wartungsaufwand erfordern.

Der Erfindung liegt die Aufgabe zugrunde, eine Vereinfachung des Standes der Technik aufzuzeigen und ein Verfahren zur Fremdstoffinspektion zu schaffen, bei welchem zweckmäßig nur ein Analysesystem, beispielsweise ein Massenspektrometer, erforderlich ist, dem die jeweilige Prüfluft einer jeden Flasche nacheinander zugeführt wird. Hierdurch soll der gesamte Aufwand einer solchen Inspektionsmaschine verringert werden. Gleichzeitig soll mit der Erfindung trotz verminderter Meßsysteme eine möglichst hohe Durchsatzleistung von zu prüfenden Flaschen sichergestellt werden.

Diese Aufgabe wird gemäß der Erfindung dadurch gelöst, daß bei einer Analyse einer vorhergehenden Flasche bereits eine vorbestimmbare Luftmenge der nachfolgenden und/oder weiteren Flasche angesaugt und innerhalb des Systems zur anschließenden unmittelbaren Zugabe zum Analysegerät in Bereitschaft gehalten und nach der Analyse der vorhergehenden Flasche dem Analysegerät zugeführt und nach deren Analyse durch Umkehr der Strömungsrichtung und Zuleiten von Spülgas aus dem System entfernt wird.

Mit einem solchen Verfahren ist der Vorteil gegeben, daß die jeweils zu überprüfende Luftmenge zumindest teilweise praktisch schon vor der eigentlichen Analyse innerhalb des System zur Verfügung steht und ohne größere Vorlaufzeiten direkt zur Analyse geleitet werden kann. Verlustzeiten, wie sie beispielsweise bei Überprüfung einer ersten Flasche, der dann folgenden Entleerung und einer nachfolgenden Ansaugung der Luft aus der nächsten Flasche entstehen, werden durch die vorgeschlagene Bevorratung der Prüfluft vermieden.

Dabei hat es sich als zweckmäßig erwiesen, daß zur Entleerung der Meßsonde als Spülgas neutrale Luft mit erhöhter Geschwindigkeit entgegen der Prüfluftansaugrichtung durch das System geführt wird.

Eine solche Umkehrung der Strömungsrichtung sowie eine erhöhte Strömungsgeschwindigkeit verbessern den Austrag von eventuellen Verschleißteilen oder Staubpartikeln innerhalb des Systems. Ferner wird vorgeschlagen, daß als Spülgas erhitzte Luft verwendet wird.

Durch die Umkehrung der Strömungsrichtung ist es einerseits möglich, innerhalb der Maschine zur Verfügung stehende erhitzte Luft auf einfache Weise entgegen der Ansaugrichtung durch das Leitungssystem zu führen. Diese erhitzte Luft hat den Vorteil, daß die Regeneration der Meßgasleitung von absorbierten Kontaminat-Molekülen infolge höherer Temperaturen erheblich beschleunigt wird. Ferner erspart die indirekte Erwärmung mit heißer Spülluft eine relativ umständliche elektrische Beheizung aller Meßgasleitungen und die Übertragung der elektrischen Leistung auf das sich drehende Karussell mit Schleifringkontakten usw. Darüberhinaus können auf diese Weise auch die in die Flaschen eintauchbaren Meßsonden beheizt werden, was mit anderen Mitteln sehr schwierig ist.

Schließlich wird vorgeschlagen, daß die Saugleistung während des Vorsaugvorganges entsprechend der Durchsatzleistung der Inspektionsmaschine anpaßbar ist.

Die Vorsaugluftmenge entspricht dabei mindestens dem Leitungsvolumen von der Meßsondenmündung bis zur Übergabestelle zum Analyse-System. Da die Maschinendrehzahl aufgrund der jeweils veränderten Produktionsbedingungen nicht konstant ist, kann die Vorsaugefördermenge der Maschinendrehzahl angepaßt werden, um insbesondere bei kleinen Durchsatzleistungen ein Leersaugen der Prüfflasche vor der eigentlichen Meßphase zu vermeiden.

Eine geeignete Inspektionsmaschine zur Durchführung des Verfahrens zur Fremdstoffinspektion von Gefäßen, insbesondere von wiederverwendbaren Kunststofflaschen, bestehend aus einem umlaufenden Meßkarussell mit daran angeordneten Meßsonden, die in die zu prüfenden Flaschen einführbar sind, und einem Analysesystem, bestehend aus mindestens einer Analysevorrichtung sowie Zuleitungen von den Meßsonden zu der Analysevorrichtung mit zwischengeschalteten Steuereinrichtungen zeichnet sich dadurch aus, daß als Steuereinrichtung ein Drehschieber vorgesehen ist, dessen drehender Teil eine der Anzahl der Meßsonden entsprechende Anzahl von Öffnungen aufweist, die mit der zugeordneten Meßsonde über Leitungen verbunden sind und dessen ortfester Teil mit einer Anzahl von Steueröffnungen ausgestattet ist, die den verschiedenen Gasströmen zugeordnet sind, wobei eine erste Steueröffnung vorgesehen ist, mit welcher ein Vorsaugen der in dem Gefäß bzw. der Flasche befindlichen Luft mindestens bis zum Drehschieber und durch eine in Rotationsrichtung des drehenden Teils davor angeordnete zweite Steueröffnung das Ansaugen dieser vorgesaugten Luft mit weiterer Luft aus dem Gefäß bzw. der Flasche in Richtung Analysesystem erfolgt, wobei in Rotationsrichtung weitere Steueröffnungen vorgesehen sind, durch welche Spülgas zur Reinigung der Leitungen zuleitbar ist.

Mit einer solchen Vorrichtung ist eine besonders vorteilhafte Durchführung des vorgenannten Verfahrens gegeben. Die zu prüfende Luftmenge kann mit dieser Vorrichtung bereits in dem System bis hin zum Drehschieber bevorratet werden und steht dann anschließend nach Überfahren der Öffnungen bzw. Steueröffnungen unmittelbar zur Weiterleitung zum Analysesystem zur Verfügung. Um die Ausmaße des Drehschiebers gering zu halten, hat es sich als zweckmäßig erwiesen, daß die Öffnungen des drehbaren Teils des Drehschiebers als mit ihrer Längsachse radial gerichtete Schlitze ausgebildet sind. Auf diese Weise kann praktisch eine Vielzahl von Schlitzen mit relativ engem Abstand aneinandergereiht werden und durch deren Länge den Durchsatzbedürfnissen angepaßt werden. Zum Einsatz von mehreren Analysevorrichtungen wird vorgeschlagen, daß die Öffnungen des drehbaren Teils des Drehschiebers in zwei konzentrisch zueinander verlaufenden Reihen angeordnet sind.

Dabei hat es sich als zweckmäßig erwiesen, daß die zugeordneten Steueröffnungen bei doppelreihiger Anordnung der Öffnungen des drehbaren Teils jeweils nur von je einer Reihe anfahrbar sind. Es ist aber auch denkbar, die Steueröffnungen für den Bereich Vorsaugen und Spülen bei doppelseitiger Anordnung der Öffnungen beider Reihen gemeinsam zu erfassen und im Bereich Ansaugen für die Analyse die Öffnungen getrennt zu erfassen.

Weitere Merkmale ergeben sich aus den verbleibenden Unteransprüchen.

Insgesamt gesehen ergibt sich mit dieser Kombination der Anordnung in Doppelreihe der Vorteil, voneinander getrennte Analysesysteme und Analysevorrichtungen vorzusehen und dadurch die Leistung der Maschine zu erhöhen.

Im nachfolgenden wird die Erfindung anhand von in den Zeichnungen dargestellten Ausführungsbeispielen näher erläutert.

In der Zeichnung zeigt:

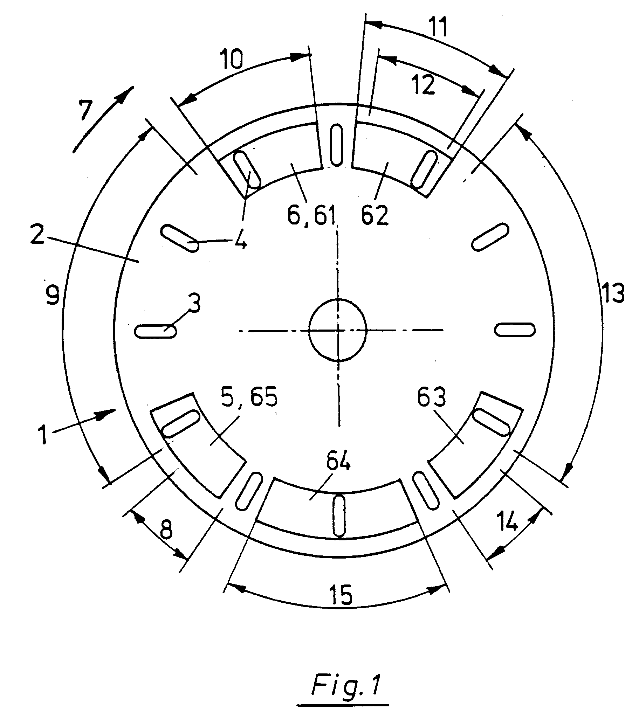

- Fig. 1: die Steuereinrichtung für ein Meßgerät,
- Fig. 2: die Steuereinrichtung für zwei voneinander getrennte Meßgeräte und
- Fig. 3: eine weitere Variante zu einer solchen Ausbildung.

Die verwendete Inspektionsmaschine ist nicht weiter dargestellt und kann aus einem umlaufenden Meßkarussell mit daran angeordneten Meßsonden bestehen. Diese sind entweder durch Hubbewegung der Meßsonden in die zu prüfenden Flaschen einführbar oder durch Anheben der Flaschen gegen die Meßsonden mit dem Flaschenhohlraum in Verbindung bringbar. Das Meßkarussell weist ferner eine Zuführungs- und Abführungseinrichtung auf, die zum Zu- und Abführen der zu prüfenden Flaschen dient. Das jeweilige Analysesystem kann ortfest innerhalb der Maschine angebracht und mit weiteren Geräten ausgestattet sein, die verschiedene Untersuchungen erlauben.

Zur Steuerung der einzelnen Verfahrensschritte und zur Trennung zwischen dem rotierenden Teil der Maschine und dem ortsfesten Analysesystem ist eine Steuereinrichtung in Form eines Drehschiebers 1 vorgesehen, dessen drehender Teil 2 entsprechend der Anzahl der Meßsonden mit Öffnungen 3 ausgestattet ist, die zweckmäßig als mit ihrer Längsachse 4 radial gerichtete Schlitze ausgebildet sind. Der ortsfeste Teil 5 des Drehschiebers 1 weist entsprechende Steueröffnungen 6 auf, die den verschiedenen Gasströmungsschritten zugeordnet sind. Die in Rotationsrichtung 7 erste Steueröffnung 61 weist einen Anschluß an eine Unterdruckpumpe auf, mit welcher ein Vorsaugen der in den Flaschen befindlichen Luft mindestens bis zum Drehschieber 1 möglich ist. Die in Rotationsrichtung danach angeordnete Steueröffnung 62 dient zum Ansaugen der Luft für die eigentliche Analyse. Auf der gegenüberliegenden Seite dieses unteren Teils des Drehschiebers 1 sind weitere Steueröffnungen 63 - 65 vorgesehen, die beispielsweise zur Vorspülung der von den Öffnungen 3 bis zur Sonde reichenden Zuleitungen, zum Hauptspülen oder auch für Zusatzspülvorgänge genutzt werden können. Die Steueröffnungen 61 - 65 sind zweckmäßig symmetrisch zueinander angeordnet, so daß eine beliebige Rotationsrichtung des Meßkarussells je nach Einsatz einer solchen Inspektionsmaschine in Flaschentransportsystemen mit Rechts- oder Linkslauf gewählt werden kann.

Verschiedene Segmente 8 - am Umfang des in den Zeichnungen dargestellten Drehschiebers 1 zeigen die einzelnen Ablaufphasen an. So zeigt beispielsweise der Abschnitt 8 den Flascheneinlauf, der Abschnitt 9 das Eintauchen der Meßsonden, der Abschnitt 10 das Vorsaugen der Flaschenluft bis zum Drehschieber 1, Abschnitt 11 das weitere Ansaugen der bis zum Drehschieber 1 in den Zuleitungen befindlichen Luftgehaltes zur eigentlichen Analysevorrichtung und der Abschnitt 12 die eigentliche Meßphase, ein weiterer Abschnitt 13, 14 das Herausziehen der Meßsonden aus den Gefäßen bzw. das Entfernen der Gefäße von den Meßsonden, den Spülabschnitt und gegebenenfalls weitere Spülabschnitte 15 z. B. mit erwärmter Druckluft usw. Die zu prüfenden Flaschen werden also zunächst im Einlaufbereich beispielsweise von heb- und senkbaren Standtellern übernommen und gegen die nicht weiter dargestellten Meßsonden geführt. Sobald die Meßsonden innerhalb des Flaschenhohlraumes eingetaucht sind, kann direkt das Vorsaugen der in der Flasche befindlichen Luft vorgenommen werden, wozu die Steueröffnung 61 mit einem Vakuumkanal in Verbindung steht. Sobald nun der Längsschlitz 4 die Steueröffnung 61 erreicht, steht das Vakuum in der Zuleitung bis zur Meßsonde an und zieht einen gewissen Teil der Flaschenluft in Richtung Drehschieber 1. Unmittelbar darauf erreicht der Längsschlitz 4 die nachfolgende Steueröffnung 62, in der ebenfalls ein Vakuum ansteht und auf diese Weise die bereits vorgespeicherte Luft und weitere aus dem Flaschenhohlraum abgesaugt und gegebenenfalls über eine Bypassleitung einer oder mehreren Analysevorrichtungen zugeleitet wird. Sobald der Längsschlitz 4 die Steueröffnung 62 überfahren hat, können die Meßsonden wieder aus der Flasche entfernt werden und diese im Bereich des Abschnittes 14 aus der Inspektionsmaschine entfernt werden. Dabei gelangt der Längsschlitz in den Bereich einer Steueröffnung 64, die beispielsweise mit heißer Druckluft oder einem anderen erhitzten Spülgas beaufschlagt ist und ein Spülen der Zuleitung von dem Drehschieber 1 bis hin zur Meßsonde veranlaßt. Eine weitere Steueröffnung 63 kann vorgesehen sein, die den Spülbereich nach Bedarf erweitert. Je nach Anlagenauslegung ist es denkbar, eine weitere Spülöffnung 65 vorzusehen, die bei entgegengesetzter Laufrichtung die Steueröffnung 63 ersetzt.

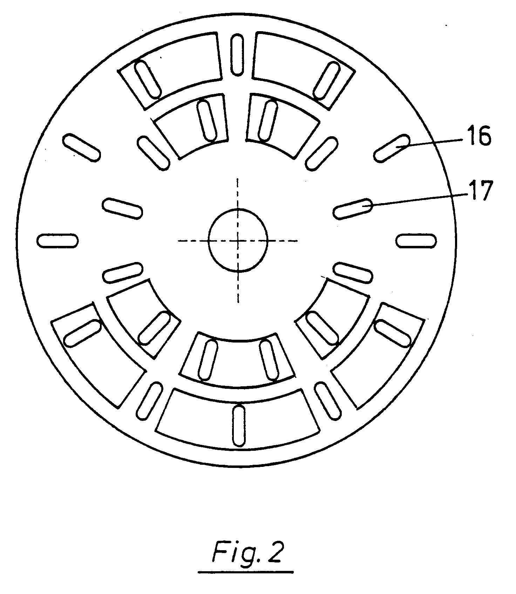

Gemäß dem in Fig. 2 dargestellten Ausführungsbeispiel sind die Öffnungen 3 des drehbaren Teils 2 des Drehschiebers 1 in zwei konzentrisch zueinander verlaufenden Reihen 16, 17 angeordnet. Ebenfalls befinden sich entsprechend angeordnete Steueröffnungen 61 - 65 in Doppelreihe in dem unteren Teil 5 des Drehschiebers 1. Auf diese Weise kann bei gleicher Drehschieberausbildung entweder eine doppelte Anzahl von Meßsonden angeschlossen oder gegebenenfalls eine Analyse mit mehreren Analysevorrichtungen vorgenommen werden, denen die eine Reihe von Steueröffnungen 61 - 65 zugeordnet ist. Die einzelnen Verfahrensschritte laufen in der Weise ab, wie sie bereits zu der einreihig ausgebildeten Drehschiebersteuerung beschrieben sind.

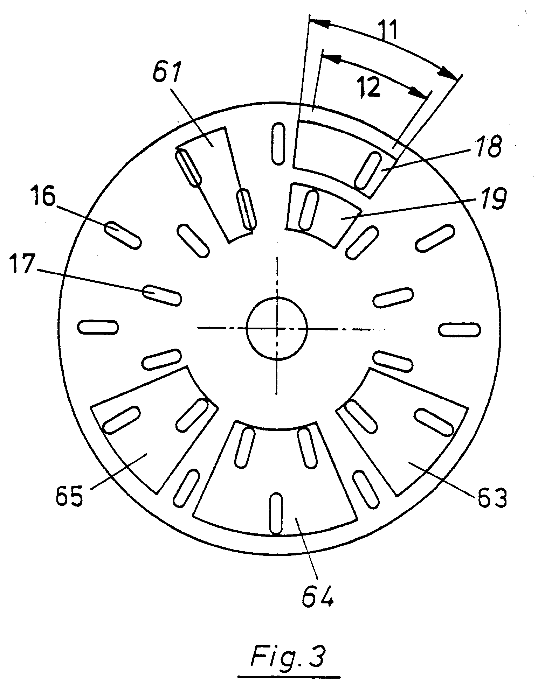

Gemäß der in Fig. 3 dargestellten Ausbildung des Drehschiebers 1 sind ebenfalls doppelreihige Schlitze 16, 17 vorgesehen, die die Steueröffnungen insbesondere für Vorsaugen 61 oder Spülen 63 - 65 gemeinsam anfahren und in den Bereichen des Abschnittes 11, 12 jeweils nur die direkt zugeordnete Steueröffnung 18, 19 überfahren, was dann ebenfalls eine Analyse mit zwei Analysevorrichtungen zuläßt.

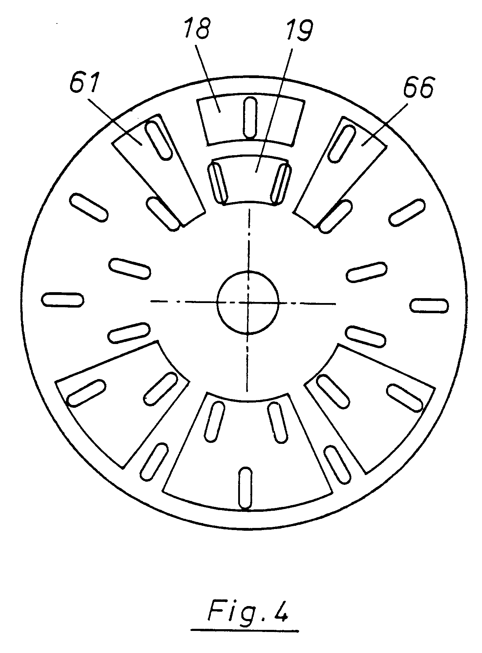

Fig. 4 zeigt einen Drehschieber für eine Maschine mit zwei Analysegeräten, wobei bei Ausfall eines Analysegerätes der Betrieb der Maschine mit dem noch intakten Analysegerät bei halber Leistung aufrecht erhalten wird.

In diesem Falle erhält der Drehschieber mit zwei Schlitzreihen gemäß Abb. 4 ein zusätzliches "Hilfs"-Steuerfenster (66) zum Messen, das beide Öffnungsreihen 3 überdeckt, jedoch so, daß immer nur aus einem Schlitz abwechselnd aus jeder Öffnungsreihe 3 angesaugt wird.

Bei Ausfall eines Analysegerätes wird die Anschlußleitung des anderen noch funktionstüchtigen Analysegerätes von dem der jeweiligen Schlitzreihe zugeordneten "Haupt"-Steuerfenster auf das zusätzliche "Hilfs"-Steuerfester 66 umgeschaltet. Da die zentrale Maschinensteuerung den Ausfall eines Meßsystems erkennt, kann die Umschaltung automatisch erfolgen.

Wenn je nach Beschaffenheit des Flaschenrücklaufgutes eine Ablagerung von z.B. zuckerhaltigen Rückständen im Drehschieber-Rohrsystem nicht ausgeschlossen werden kann, ermöglicht eine Spülvorrichtung bei regelmäßiger Anwendung einen störungsfreien Betrieb.

Der Spülvorgang mit temperiertem Wasser wird bei laufender Maschine ohne Flaschen vorgenommen. Hierzu werden die Rohrleitung zum Analysegerät und ggf. auch die Vorsauge- und Spülluftleitungen durch Magnetventile abgesperrt. Vor diesen Absperrungen wird temperiertes Wasser hineingedrückt, so daß es rückwärts durch den Drehschieber und die Sondenrohre fließt und diese von Ablagerungen befreit.

Nach der Reinigung wird die Maschine zur Trocknung von Restflüssigkeit eine zeitlang auf Spülung mit heißer Luft geschaltet, wobei jedoch die Heißluft in alle noch abgesperrten Leitungen eingeleitet wird.

Zur Schmierung der Drehschiebergleitflächen mit einem geeigneten Medium unter Druck befinden sich in tragfähigen Stellen des Drehschiebers, in deren Nähe sich keine Steueröffnungen befinden, Bohrungen, durch die unter Druck ein geeignetes Medium, vorzugsweise Luft eingeleitet wird. Der sich aufbauende "Schmier"-Film ermöglicht einen praktisch verschleißfreien Betrieb des Drehschiebers, wodurch aufwendige Beschichtungen der Gleitflächen, z.B. mit Hartmetall usw., entfallen. Voraussetzung für diese Schmierung ist, daß die Verdünnung des zu analysierenden Gases mit dem Schmiermedium von untergeordneter Größenordnung ist, was durch eine geeignete Dimensionierung des Drehschiebers technisch realisierbar ist.

Als Analysesystem kann ein geeignetes Massenspektrometer oder ein anderes System verwendet werden, die zentral angeordnet und ansteuerbar sind.

## Patentansprüche

1. Verfahren zur Fremdstoffinspektion von Gefäßen, insbesondere von wiederverwendbaren Kunststoffflaschen, die in einer Inspektionsmaschine nacheinander einer Überprüfung unterzogen werden, wobei ein Teil der in dem Gefäß vorhandenen Luft von in den Gefäßhohlraum einführbaren Meßsonden angesaugt und einer Analyse unterworfen wird, **dadurch gekennzeichnet,** daß bei einer Analyse einer vorhergehenden Flasche bereits eine vorbestimmbare Luftmenge der nachfolgenden und/oder weiteren Flasche angesaugt und innerhalb des Systems zur anschließenden unmittelbaren Zugabe zum Analysegerät in Bereitschaft gehalten und nach der Analyse der vorhergehenden Flasche dem Analysegerät zugeführt und nach deren Analyse durch Umkehr der Strömungsrichtung und Zuleiten von Spülgas aus dem System entfernt wird.

2. Verfahren nach Anspruch 1, **dadurch gekennzeichnet,** daß als Spülgas neutrale Luft mit erhöhter Geschwindigkeit entgegen der Prüfluftansaugrichtung durch das System geführt wird.

3. Verfahren nach Anspruch 1, **dadurch gekennzeichnet,** daß als Spülgas erhitzte Luft verwendet wird.

4. Verfahren nach Anspruch 1, **dadurch gekennzeichnet,** daß die Saugleistung während des Vorsaugvorganges entsprechend der Durchsatzleistung der Inspektionsmaschine anpaßbar ist.

5. Inspektionsmaschine zur Fremdstoffinspektion von Gefäßen, insbesondere von wiederverwendbaren Kunststoffflaschen, bestehend aus einem umlaufenden Meßkarussell mit daran angeordneten Meßsonden, die in die zu prüfenden Flaschen einführbar sind, und einem Analysesystem, bestehend aus mindestens einer Analysevorrichtung sowie Zuleitungen von den Meßsonden zu der Analysevorrichtung mit zwischengeschalteten Steuereinrichtungen, insbesondere zur Durchführung des Verfahrens nach den Ansprüchen 1 - 4, **dadurch gekennzeichnet,** daß als Steuereinrichtung ein Drehschieber (1) vorgesehen ist, dessen drehender Teil (2) eine der Anzahl der Meßsonden entsprechende Anzahl von Öffnungen (3) aufweist, die mit der zugeordneten Meßsonde über Leitungen verbunden sind und dessen ortsfester Teil (5) mit einer Anzahl von Steueröffnungen (6) ausgestattet ist, die den verschiedenen Gasströmen zugeordnet sind, wobei eine erste Steueröffnung (61) vorgesehen ist, mit welcher ein Vorsaugen der in dem Gefäß bzw. der Flasche befindlichen Luft mindestens bis zum Drehschieber (1) und durch eine in Rotationsrichtung des drehenden Teils (2) davor angeordnete zweite Steueröffnung (62) das Ansaugen dieser vorgesaugten Luft mit weiterer Luft aus dem Gefäß bzw. der Flasche in Richtung Analysesystem erfolgt, und in der Rotationsrichtung weitere Steueröffnungen (63 - 65) vorgesehen sind, durch welche Spülgas zur Reinigung der Leitungen zuleitbar ist.

6. Inspektionsmaschine nach Anspruch 5, **dadurch gekennzeichnet,** daß die Öffnungen des drehbaren Teils (2) des Drehschiebers (1) als mit ihrer Längsachse radial gerichtete Schlitze (3, 4) ausgebildet sind.

7. Inspektionsmaschine nach einem oder mehreren der vorhergehenden Ansprüche 5 und 6, **dadurch gekennzeichnet,** daß die Öffnungen (16, 17) des drehbaren Teils (2) des Drehschiebers (1) in zwei konzentrisch zueinander verlaufenden Reihen angeordnet sind.

8. Inspektionsmaschine nach den vorhergehenden Ansprüchen 5 bis 7, **dadurch gekennzeichnet,** daß die zugeordneten Steueröffnungen (18, 19) bei doppelreihiger Anordnung der Öffnungen (16, 17) des drehbaren Teils (2) jeweils nur von je einer Reihe anfahrbar sind.

9. Inspektionsmaschine nach Anspruch 5, **dadurch gekennzeichnet,** daß die Steueröffnungen (63 - 65) für den Bereich Vorsaugen und Spülen bei doppelseitiger Anordnung der Öffnungen (16, 17) beide Reihen gemeinsam erfassen und im Bereich Ansaugen für die Analyse die Öffnungen (18, 19) getrennt erfassen.

10. Inspektionsmaschine nach einem oder mehreren der vorhergehenden Ansprüche 5 bis 9, **dadurch gekennzeichnet,** daß eine zusätzliche Hilfssteueröffnung (66) vorgesehen ist, die bei mehrreihiger Schlitzanordnung die zugeordneten Schlitzreihen (16, 17) überdeckt.

11. Inspektionsmaschine nach einem oder mehreren der vorhergehenden Ansprüche 5 bis 10, **dadurch gekennzeichnet,** daß zur Spülung des Systems eine Spülflüssigkeit entgegen der Ansaugrichtung geleitet wird und die Analyseleitungen absperrbar sind.

12. Inspektionsmaschine nach einem oder mehreren der vorhergehenden Ansprüche 5 bis 11, **dadurch gekennzeichnet,** daß zur Schmierung des Drehschiebers (1) zwischen den gegeneinander liegenden Laufflächen Druckluft und/oder ein anderes geeignetes Schmiermedium zuleitbar ist.

## Claims

1. Method of checking for impurities in vessels, more especially in re-usable plastics material bottles, which are successively subjected to a checking process in an inspection machine, a portion of the air in the vessel being drawn-in by measuring probes, which are insertable in the vessel cavity, and being subjected to an analysis, characterised in that, during an analysis of a preceding bottle, a predeterminable quantity of air from the subsequent and/or additional bottle is already drawn-in and kept in readiness internally of the system for the subsequent direct addition to the analysing device, such air being supplied to the analysing device after the analysis of the preceding bottle and being removed from the system, after said bottle has been analysed, by reversing the direction of flow and by supplying scavaging gas.

2. Method according to claim 1, characterised in that neutral air is guided as scavaging gas through the system at a high speed in a direction in opposition to the direction in which the testing air is drawn-in by suction.

3. Method according to claim 1, characterised in that heated air is used as the scavaging gas.

4. Method according to claim 1, characterised in that the suction power is adaptable during the preliminary suction process according to the throughput capacity of the inspection machine.

5. Inspection machine for inspecting foreign bodies invessels, more especially in re-usable plastics material bottles, comprising a rotatable measuring carrousel having measuring probes disposed thereon, which probes are insertable in the bottles to be tested, and an analysing system, comprising at least one analysing device as well as supply lines extending from the measuring probes to the analysing device with control means interposed therebetween, more especially for accomplishing the method according to claims 1 - 4, characterised in that a rotary slide (1) is provided as the control means, the rotatable member (2) of said slide having a number of apertures (3) corresponding to the number of measuring probes, which apertures are connected to the associated measuring probe via lines, and the stationary member (5) of said slide being provided with a number of ports (6) which are associated with the various gas streams, a first port (61) being provided, by means of which a preliminary suction of the air situated in the vessel, or respectively in the bottle, is effected at least up to the rotary slide (1), and this pre-sucked air is drawn-in through a second port (62), which is disposed upstream thereof when viewed with respect to the rotational direction of the rotatable member (2), with additional air from the vessel, or respectively from the bottle, in the direction of the analysing system, and additional ports (63 - 65) are provided in the rotational direction, through which ports scavaging gas is suppliable to clean the lines.

6. Inspection machine according to claim 5, characterised in that the apertures of the rotatable member (2) of the rotary slide (1) are configured as slots (3, 4), which are radially orientated with their longitudinal axis.

7. Inspection machine according to one or more of the preceding claims 5 and 6, characterised in that the apertures (16, 17) of the rotatable member (2) of the rotary slide (1) are disposed in two rows which extend concentrically relative to each other.

8. Inspection machine according to the preceding claims 5 to 7, characterised in that each of the associated ports (18, 19) can only be provided in one row when the apertures (16, 17) of the rotatable member (2) are disposed in two rows.

9. Inspection machine according to claim 5, characterised in that, when the apertures (16, 17) are disposed on both sides, the ports (63 - 65) for the "preliminary suction" and "rinsing" section determine both rows jointly and determine the apertures (18, 19) separately in the "drawing-in" section for the analysis.

10. Inspection machine according to one or more of the preceding claims 5 to 9, characterised in that an additional auxiliary port (66) is provided which, when a plurality of rows of slots are provided, covers the associated rows of slots (16, 17).

11. Inspection machine according to one or more of the preceding claims 5 to 10, characterised in that a rinsing fluid is conducted in a direction in opposition to the drawing-in direction to rinse the system, and the analysing lines can be closed.

12. Inspection machine according to one or more of the preceding claims 5 to 11, characterised in that compressed air and/or another suitable lubricating medium is suppliable for lubricating the rotary slide (1) between the oppositely situated bearing faces.

## Revendications

1. Procédé d'inspection de corps étrangers dans des récipients, en particulier dans des bouteilles en matière plastique réutilisables, qui sont soumis l'un après l'autre dans une machine d'inspection à une vérification, une partie de l'air contenu dans le récipient étant aspiré par des sondes de mesure à introduire dans la cavité du récipient et étant soumise à une analyse, caractérisé en ce que lors d'une analyse d'une bouteille précédente, une quantité d'air prédéterminable de la bouteille suivante et/ou d'une autre bouteille est aspirée et est mise en attente à l'intérieur du système pour l'ajout immédiat suivant à l'appareil d'analyse et après l'analyse de la bouteille précédente, est envoyée à l'appareil d'analyse, et après son analyse, est éliminée du système, par inversion du sens d'écoulement et envoi de gaz de lavage.

2. Procédé selon la revendication 1, caractérisé en ce que comme gaz de lavage on envoie de l'air neutre à travers le système, à vitesse accrue, dans le sens contraire au sens d'aspiration de l'air d'essai.

3. Procédé selon la revendication 1, caractérisé en ce qu'on utilise de l'air chauffé comme gaz de lavage.

4. Procédé selon la revendication 1, caractérisé en ce que le volume aspiré pendant l'opération d'aspiration préalable peut être adapté en fonction du débit de la machine d'inspection.

5. Machine d'inspection pour l'inspection de corps étrangers dans des récipients, en particulier dans des bouteilles en matière plastique réutilisables, constituée d'un carrousel de mesure tournant avec sondes de mesure placées sur celui-ci, qui peuvent être introduites dans les bouteilles à contrôler, et d'un système d'analyse, constitué d'au moins un dispositif d'analyse ainsi que de conduites d'arrivée en provenance des sondes de mesure, dirigées vers le dispositif d'analyse, avec dispositifs de commande insérés, en particulier pour la mise en oeuvre du procédé selon les revendications 1 à 4, caractérisée en ce que comme dispositif de commande il est prévu un tiroir rotatif (1), dont la partie tournante (2) comporte un nombre d'ouvertures (3) correspondant au nombre de sondes de mesure, qui sont reliées avec la sonde de mesure associée, par des lignes et dont la partie fixe (5) est équipée d'un certain nombre d'ouvertures de commande (6), qui sont associées aux différents courants de gaz, une première ouverture de commande (6) étant prévue par laquelle s'effectue une aspiration préalable de l'air se trouvant dans le récipient ou la bouteille, au moins jusqu'au tiroir rotatif (1) tandis que par une deuxième ouverture de commande (62), placée devant dans le sens de rotation de la partie tournante (2), a lieu l'aspiration de cet air pré-aspiré avec une autre quantité d'air, du récipient ou de la bouteille en direction du système d'analyse, et dans le sens de rotation sont prévues d'autres ouvertures de commande (63 à 65), par lesquelles du gaz de lavage peut être envoyé pour le nettoyage des conduites.

6. Machine d'inspection selon la revendication 5, caractérisée en ce que les ouvertures de la partie tournante (2) du tiroir rotatif (1) sont conformées en fentes (3, 4) dans l'axe longitudinal est dirigé radialement.

7. Machine d'inspection selon une ou plusieurs des revendications précédentes 5 et 6, caractérisée en ce que les ouvertures (16, 17) de la partie tournante (2) du tiroir rotatif (1) sont disposées en deux rangées concentriques l'une à l'autre.

8. Machine d'inspection selon les revendications précédentes 5 à 7, caractérisée en ce que dans le cas d'une disposition à double rangée des ouvertures (16, 17) de la partie tournante (2), les ouvertures de commande (18, 19) associées ne peuvent être touchées chacune que par une rangée.

9. Machine d'inspection selon la revendication 5, caractérisée en ce que dans le cas d'une disposition bilatérale des ouvertures (16, 17), les ouvertures de commande (63 à 65) pour le domaine aspiration préalable et lavage, touchent conjointement les deux rangées et dans le domaine aspiration pour l'analyse, touchent séparément les ouvertures (18, 19).

10. Machine d'inspection selon une ou plusieurs des revendications précédentes 5 à 9, caractérisée en ce qu'il est prévu une ouverture de commande auxiliaire (66) supplémentaire, qui en cas de disposition de fentes sur plusieurs rangées, recouvre les rangées de fentes (16, 17) associées.

11. Machine d'inspection selon une ou plusieurs des revendications précédentes 5 à 10, caractérisée en ce que pour le lavage du système, un liquide de lavage est envoyé dans le sens contraire au sens d'aspiration et les conduites d'analyse peuvent être obturées.

12. Machine d'inspection selon une ou plusieurs des revendications précédentes 5 à 11, caractérisée en ce que pour la lubrification du tiroir rotatif (1), de l'air comprimé et/ou un autre produit lubrifiant approprié peut être envoyé entre les surfaces de glissement s'appuyant l'une contre l'autre.
